# EUROPEAN PATENT APPLICATION

(11) **EP 2 985 024 A1**
(43) Date of publication of application: **17.02.2016**
(21) Application number: 14763917.3
(22) Date of filing: 20.02.2014
(51) Int. Cl.: A61K 31/495, A61P 19/00, A61P 43/00

(54) **THERAPEUTIC AGENT FOR SYSTEMIC BONE DISEASE AND USE THEREOF**

(30) Priority: 10.03.2013 JP 2013047426
(71) Applicant: National University Corporation Nagoya University, Nagoya-shi, Aichi 464-8601 (JP)
(72) Inventor: OHNO, Kinji, Nagoya-shi Aichi 464-8601 (JP); ISHIGURO, Naoki, Nagoya-shi Aichi 464-8601 (JP); KITO, Hiroshi, Nagoya-shi Aichi 464-8601 (JP); MATSUSHITA, Masaki, Nagoya-shi Aichi 464-8601 (JP)
(74) Representative: Steinecke, Peter
(86) International application number: PCT/JP2014/054023
(87) International publication number: WO 2014/141847

(57) **Abstract**

The object is to provide a novel therapeutic strategy achieving high therapeutic effect on a skeletal dysplasia caused by excessive activation of FGFR3, particularly achondroplasia and hypochondroplasia. Provided is a therapeutic agent for a skeletal dysplasia, including meclizine or its pharmaceutically acceptable salt as an active ingredient.

## Description

### TECHNICAL FIELD

The present invention relates to a therapeutic agent for skeletal dysplasia and a use thereof. The therapeutic agent of the present invention for skeletal dysplasia is applicable to the treatment of achondroplasia, hypochondroplasia, thanatophoric dysplasia, Crouzon disease, acromesomelic dysplasias, and severe achondroplasia with developmental delay and acanthosis nigricans (SADDAN). The present application claims the benefit of priority from prior Japanese Patent Application No. 2013-47426, filed on March 10, 2013, the entire contents of which are incorporated herein by reference.

### BACKGROUND ART

Achondroplasia (ACH) and hypochondroplasia (HCH) are developed by a constantly active mutant of fibroblast growth factor receptor 3 (FGFR3), which is a negative regulator of bone growth. In addition, involvement of FGFR3 in short stature including idiopathic dwarfism is also shown. Achondroplasia is a bone growth disorder which causes not only short stature but also serious complications such as spinal canal stenosis and foramen magnum stenosis. There is no radical therapy for suppressing activity of FGFR3 in ACH/HCH. As supportive treatment for short stature, growth hormone treatment and bone lengthening are carried out in medical and surgical manners.

Recently, small chemical compounds that antagonize FGFR3 signaling have been identified. Toxicological profiles of these compounds, however, remain mostly unresolved (Non Patent Literatures 1-3). The C-type natriuretic peptide (CNP) is a potent antagonist of FGFR3 signaling that alleviates short-limbed phenotype of ACH/HCH through its inhibition of the FGFR3-MAPK pathway (Non Patent Literatures 4 and 5). CNP has a very short half-life and continuous intravenous infusion is required for in vivo experiments (Non Patent Literature 6). The CNP analog with extended half-life, BMN 111, has recently been developed and significant recovery of bone growth was demonstrated in ACH/HCH mice by subcutaneous administration (Non Patent Literature 7).

Various new therapeutic strategies for achondroplasia are reported (for example, see Patent Literatures 1 to 3).

### PRIOR ART LITERATURE

### PATENT LITERATURE

Patent Literature 1: Japanese Unexamined Patent Application Publication (Translation of PCT Application) No. 11-507828
Patent Literature 2: Japanese Unexamined Patent Application Publication (Translation of PCT Application) No. 2011-504506
Patent Literature 3: Japanese Unexamined Patent Application Publication No. 2003-104908

### NON- PATENT LITERATURE

Non-Patent Literature 1:
   Krejci, P., Murakami, S., Prochazkova, J., Trantirek, L., Chlebova, K., Ouyang, Z., Aklian, A., Smutny, J., Bryja, V., Kozubik, A. et al. (2010) NF449 is a novel inhibitor of fibroblast growt h factor receptor 3 (FGFR3) signaling active in chondrocytes and multiple myeloma cells. J. Biol. Chem., 285, 20644-20653.
Non-Patent Literature 2:
   Jin, M., Yu, Y., Qi, H., Xie, Y., Su, N., Wang, X., Tan, Q., Luo, F., Zhu, Y., Wang, Q. et al. (20 12) A novel FGFR3-binding peptide inhibits FGFR3 signaling and reverses the lethal phenoty pe of mice mimicking human thanatophoric dysplasia. Hum. Mol. Genet., 21, 5443-5455.
Non-Patent Literature 3:
   Jonquoy, A., Mugniery, E., Benoist-Lasselin, C., Kaci, N., Le Corre, L., Barbault, F., Girard, A.L., Le Merrer, Y., Busca, P., Schibler, L. et al. (2012) A novel tyrosine kinase inhibitor resto res chondrocyte differentiation and promotes bone growth in a gain-of-function Fgfr3 mouse model. Hum. Mol. Genet., 21, 841-851.
Non-Patent Literature 4:
   Krejci, P., Masri, B., Fontaine, V., Mekikian, P.B., Weis, M., Prats, H. and Wilcox, W.R. (2005 ) Interaction of fibroblast growth factor and C-natriuretic peptide signaling in regulation of ch ondrocyte proliferation and extracellular matrix homeostasis. J. Cell Sci., 118, 5089-5100.
Non-Patent Literature 5:
   Yasoda, A., Komatsu, Y., Chusho, H., Miyazawa, T., Ozasa, A., Miura, M., Kurihara, T., Rogi, T., Tanaka, S., Suda, M. et al. (2004) Overexpression of CNP in chondrocytes rescues achond roplasia through a MAPK-dependent pathway. Nat. Med., 10, 80-86.
Non-Patent Literature 6:
   Yasoda, A., Kitamura, H., Fujii, T., Kondo, E., Murao, N., Miura, M., Kanamoto, N., Komatsu , Y., Arai, H. and Nakao, K. (2009) Systemic administration of C-type natriuretic peptide as a novel therapeutic strategy for skeletal dysplasias. Endocrinology, 150, 3138-3144.
Non-Patent Literature 7:
   Lorget, F., Kaci, N., Peng, J., Benoist-Lasselin, C., Mugniery, E., Oppeneer, T., Wendt, D.J., B ell, S.M., Bullens, S., Bunting, S. et al. (2012) Evaluation of the therapeutic potential of a CN P analog in a Fgfr3 mouse model recapitulating achondroplasia. Am J Hum Genet, 91, 1108-1 114.

### SUMMARY OF THE INVENTION

### PROBLEM TO BE SOLVED BY THE INVENTION

Growth hormone treatment utilizes the action of insulin-like growth factor-1 (IGF-1) extending epiphyseal cartilage. However, achondroplasia is not generally accompanied by the decrease of activity of IGF-1, so that expected effect will not be achieved. In addition, the effect is shown only immediately after initiation of the treatment, but the effect slowly diminishes. On the other hand, bone lengthening regenerates the bone by gradually extending the callus formed by osteotomy, but this method has problems that maturation of the extended callus requires a long period of time, whereby complications such as pin tract infection and joint contracture increase. Therefore, the present invention is intended to provide a novel therapeutic strategy achieving high therapeutic effect on a skeletal dysplasia (particularly achondroplasia and hypochondroplasia) caused by excessive activation of FGFR3.

### MEANS FOR SOLVING PROBLEM

The present inventors focused on activation of FGFR3 signaling, and tried to find a low molecular weight compound specifically suppressing activation of FGFR3 signaling from approved drugs. Firstly, a screening was carried out in the experimental system that FGFR3 signaling is activated by the addition of the fibroblast growth factor 2 (FGF2) to rat chondrosarcoma (RCS) cells causing suppression of cell proliferation and loss of extracellular matrix. Specifically, 1,186 approved drugs (Prestwick Chemicals) were added to the mixture of the RCS cells and FGF2, and the effect was studied. As a result of the detailed study, amazingly, meclizine, which is frequently used as a motion sickness drug having antihistaminic action, rescued the suppression of cell proliferation and the loss of extracellular matrix in a concentration-dependent manner. In addition, the introduction of the mutated FGFR3 into human chondrosarcoma cells and ATDC5 cells as cartilage cells caused the suppression of cell proliferation and the loss of extracellular matrix, but meclizine rescued these actions. Furthermore, the longitudinal growth of bone was suppressed when the tibia bone of a mouse on day 16.5 of intrauterine life was cultured in the presence of FGF2, but meclizine inhibited the action of suppressing the longitudinal growth.

As described above, the experiments using cartilaginous cells and an organ culture of bones proved the effectiveness of meclizine, or that meclizine suppresses FGFR3 signaling, and shows efficacy. The use of meclizine allows the establishment of a radical therapy for various skeletal dysplasias mainly or partly caused by excessive activation of FGFR3 (in particular, diseases with short stature, such as achondroplasia, hypochondroplasia, thanatophoric dysplasia, Crouzon disease, acromesomelic dysplasias, and severe achondroplasia with developmental delay and acanthosis nigricans (SADDAN)). On the other hand, FGFR3 is always expressed as a negative regulator in the extension of normal epiphyseal cartilage. Therefore, meclizine is regarded as effective also as a body growth promoter in a normal subject. In actual, oral administration of meclizine to a normal mouse achieved growth effect on the body length and extremity length. More specifically, growth effect of meclizine was confirmed by the animal experiment. On the basis of these results, meclizine is regarded as effective also in the treatment/prevention of pituitary dwarfism not accompanied by epiphyseal closing, short stature in Turner syndrome, short stature in Prader-Willi syndrome, short stature with growth hormone deficiency (GHD), and short stature with small-for-gestational age (SGA).

The present invention described below is mainly based on the above-described findings and observations.
[1] A therapeutic agent for a skeletal dysplasia caused by excessive activation of fibroblast growth factor receptor 3 (FGFR3), the therapeutic agent including meclizine or its pharmaceutically acceptable salt as an active ingredient.
[2] The therapeutic agent of [1], wherein the skeletal dysplasia is a disease selected from the group consisting of achondroplasia, hypochondroplasia, thanatophoric dysplasia, Crouzon disease, acromesomelic dysplasias, and severe achondroplasia with developmental delay and acanthosis nigricans (SADDAN).
[3] The therapeutic agent of [1] or [2], wherein the active ingredient is meclizine hydrochloride.
[4] A therapy for a skeletal dysplasia, including administering meclizine or its pharmaceutically acceptable salt to a patient with a skeletal dysplasia caused by excessive activation of fibroblast growth factor receptor 3 (FGFR3) in a pharmaceutically effective dose.
[5] A body growth promoter including meclizine or its pharmaceutically acceptable salt as an active ingredient.
[6] A body growth promoting composition including the body growth promoter of [5].
[7] The body growth promoting composition of [6], which is a medicine, quasi drug, or food.

### BRIEF DESCRIPTION OF THE DRAWING

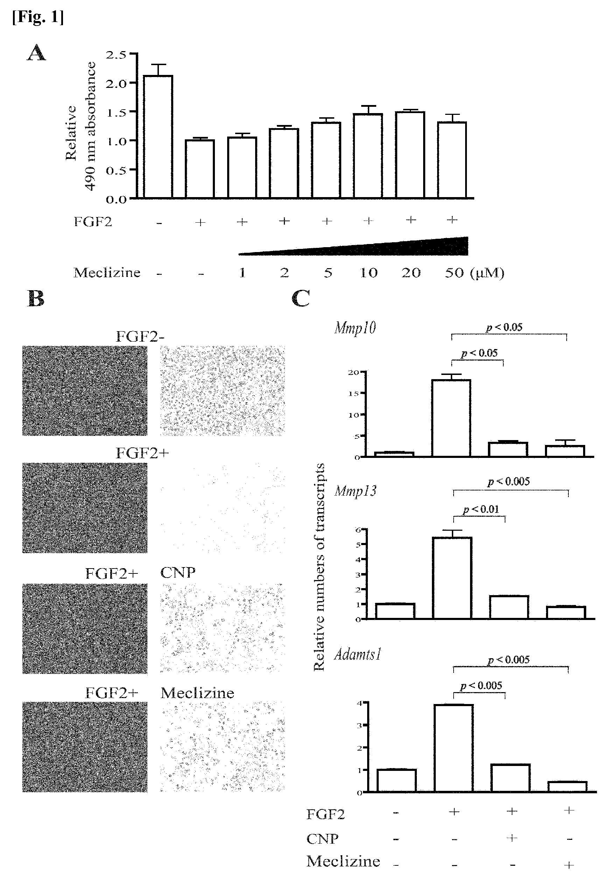

Fig. 1. Meclizine promotes chondrocyte proliferation and rescues loss of extracellular matrix in FGF2-treated RCS cells. (**A**) RCS cells were treated with 5 ng/ml FGF2 and increasing concentrations of meclizine was added for 48 hours. Cell growth was quantified using MTS assay. Data are presented as the mean and SD of 490-nm absorbance normalized for that without meclizine (n = 8). Meclizine rescued the FGF2-mediated growth arrest of RCS cells in a dose-dependent manner. Meclizine showed slight toxicity at 50 µM. (B) Meclizine rescued FGF2-mediated alteration of cellular shape and loss of extracellular matrix. RCS cells were treated with 5 ng/ml FGF2 alone or in the presence of 0.2 µM CNP or 20 µM meclizine for 72 hours, and cartilage-like sulfated proteoglycan matrix was stained by Alcian blue on the right column. Growing RCS cells were round shaped and produced abundant cartilage-like sulfated proteoglycan matrix in the absence of FGF2. These chondrocytic phenotypes were almost completely lost by FGF2 treatment. In the RCS cells treated with FGF2 together with CNP or meclizine, the cellular shape remained round shaped and the intensity of Alcian blue staining approximated that of FGF2-negative cells. (C) Meclizine inhibited mRNA expression of matrix metalloproteinases in FGF2-treated RCS cells. Cells were treated with FGF2 and either CNP or meclizine for four hours and mRNAs were quantified by real-time RT-PCR. Expression levels of *Mmp10, Mmp13,* and *Adamts1* are presented as the mean and SD normalized for that of the cells without FGF2 treatment (n = 3). FGF2-mediated increases of *Mmp10, Mmp13,* and *Adamts1* mRNA were antagonized by CNP and meclizine.

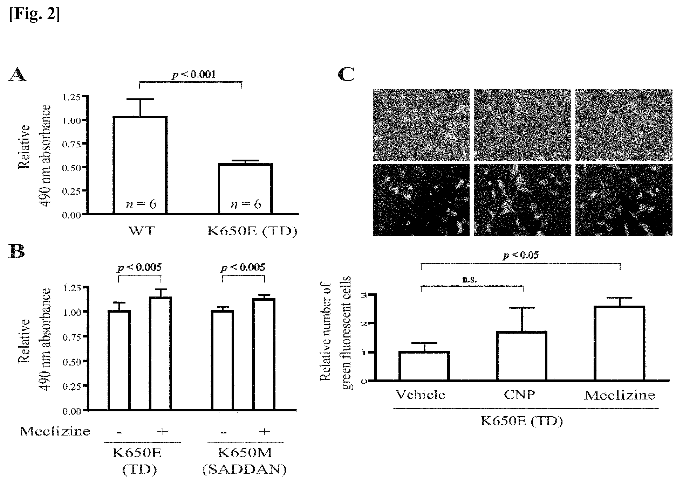

Fig. 2. Meclizine rescued abnormally suppressed proliferation of HCS-2/8 chondrocytes infected with lentivirus expressing FGFR3-K650E and -K650M. (A) Proliferation of HCS-2/8 cells expressing either FGFR3-WT (wild-type) or FGFR3-K650E (TD) was quantified using MTS assay at 48 hours after seeding. The growth of HCS-2/8 expressing FGFR3-K650E was significantly lower than that of FGFR3-WT (*p* < 0.001). The mean and SD are plotted. (B) HCS-2/8 cells expressing either FGFR3-K650E (TD) or FGFR3-K650M (SADDAN) were cultured with meclizine for 48 hours and the proliferation was quantified by MTS assay. Data are presented as the mean and SD normalized for that without meclizine (*n* = 12). Meclizine inhibited growth arrest driven by the TD and SADDAN mutants. (C) K650E-mediated growth inhibition visualized by Venus fluorescence expressed by the infected lentivirus is also rescued by CNP and meclizine. The upper panels show that the transfection efficiency is more than 90%. Both CNP and meclizine increased the integrated Venus signal intensities. Data are presented as the mean and SD normalized for that of vehicle (*n* = 3).

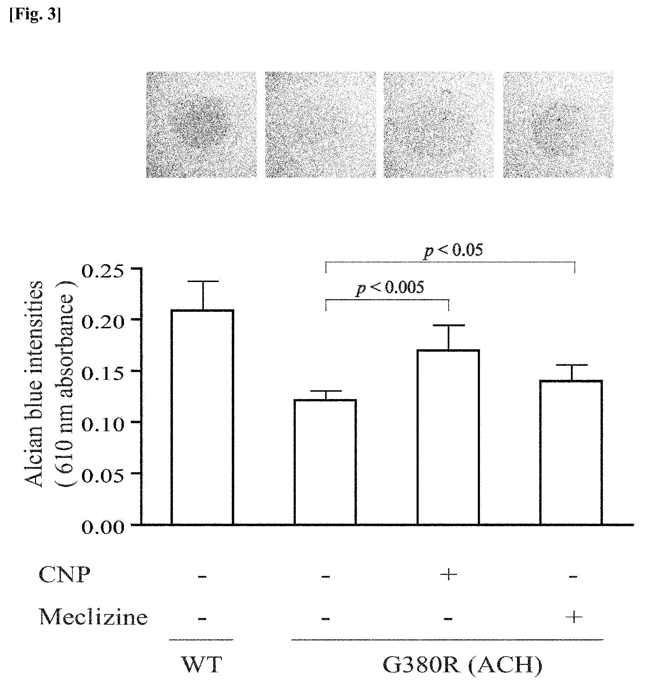

Fig. 3. Meclizine rescued abnormal inhibition of chondrocyte differentiation of ATDC5 cells infected with lentivirus expressing FGFR3-G380R (ACH) in micromass culture. ATDC5 cells in micromass culture were treated with CNP or meclizine for six days and were stained with Alcian blue. After staining, cells were lysed in 200 µl of 6 M guanidine HCl and 610-nm absorbance was quantified. The data are presented as the mean and SD (*n* = 6). CNP and meclizine increased the Alcian blue staining of ATDC5 cells expressing FGFR3-G380R in micromass culture.

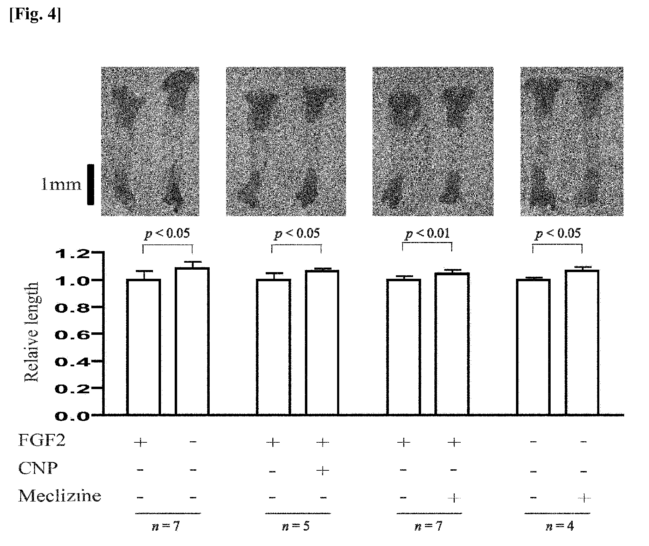

Fig. 4. Meclizine increased the longitudinal length of embryonic tibiae with or without FGF2 treatment in bone explant culture. Tibia sections were stained with hematoxylin-eosin and Alcian blue on day 6 of explant culture. Bilateral tibiae of the same individual are shown side by side. Longitudinal bone lengths are normalized for that of untreated contralateral tibia, and the mean and SD are indicated. FGF2 causes inhibition of longitudinal growth. In the presence of FGF2, CNP and meclizine significantly increased the longitudinal length of embryonic tibiae. Even without FGF2, meclizine significantly improved the growth of embryonic tibiae.

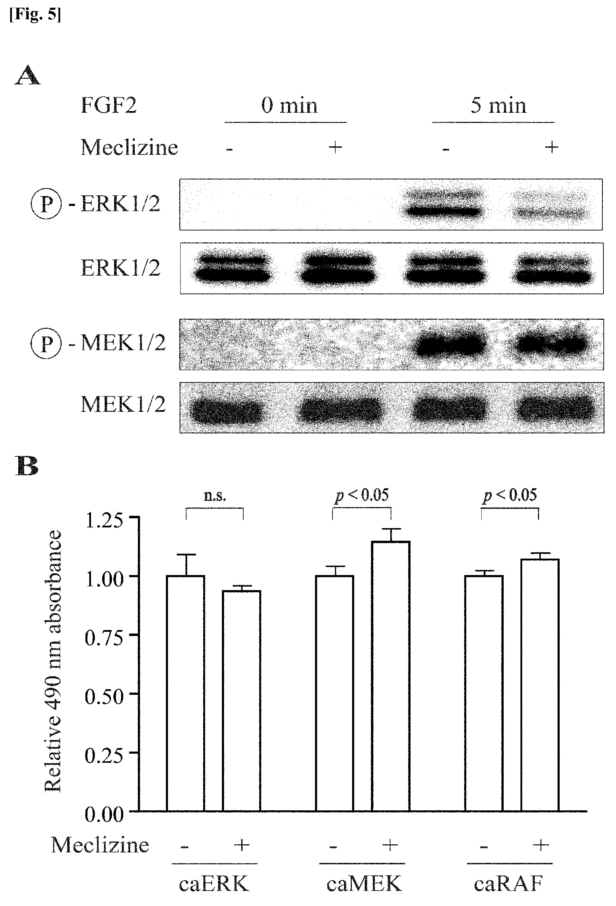

Fig. 5. Meclizine attenuates FGFR3-mediated ERK phosphorylation in FGF2-treated RCS cells. (**A**) RCS cells were pretreated with meclizine for 30 minutes before adding 5 ng/ml FGF2 and the levels of ERK and MEK phosphorylation were determined by Western blotting. As a loading control, the membranes were reprobed with antibodies against MEK and ERK. Meclizine suppressed FGF2-mediated ERK phosphorylation but not MEK phosphorylation at five minutes of FGF2 treatment. (**B**) RCS cells were infected by lentivirus expressing constitutively active (ca) ERK, MEK, and RAF mutants. Cells were treated with meclizine and their proliferation potencies were quantified using MTS assay. The 490-nm absorbance is normalized for that without meclizine treatment and the mean and SD are presented (n = 3). Meclizine rescued caMEK- and caRAF-mediated growth arrest, but had no effect on caERK-mediated growth arrest.

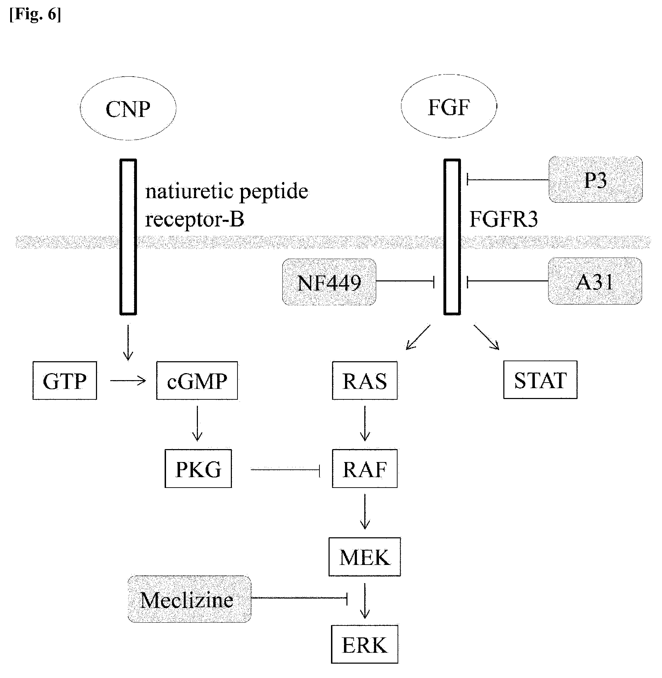

Fig. 6. FGFR3 signal transduction in cartilage and mechanisms of FGFR3 inhibitors. Mitogen-activated protein kinase (MAPK) and signal transducers and activators of transcription (STAT) signals negatively regulate chondrocyte proliferation and differentiation. MAPK signaling includes sequential stimulation of a signaling cascade involving RAS, RAF, MEK, and ERK. CNP binding to natriuretic peptide receptor-B induces the generation of the second messenger cGMP, which activates PKG and leads to attenuation of the MAPK pathway through RAF. NF449 (Reference 14), A31 (Reference 16), and P3 (Reference 15) are recently identified FGFR3 inhibitors. NF449 and A31 have inhibitory effects on FGFR3 kinase activity. P3 has an affinity for extracellular domain of FGFR3. Meclizine suppresses ERK phosphorylation.

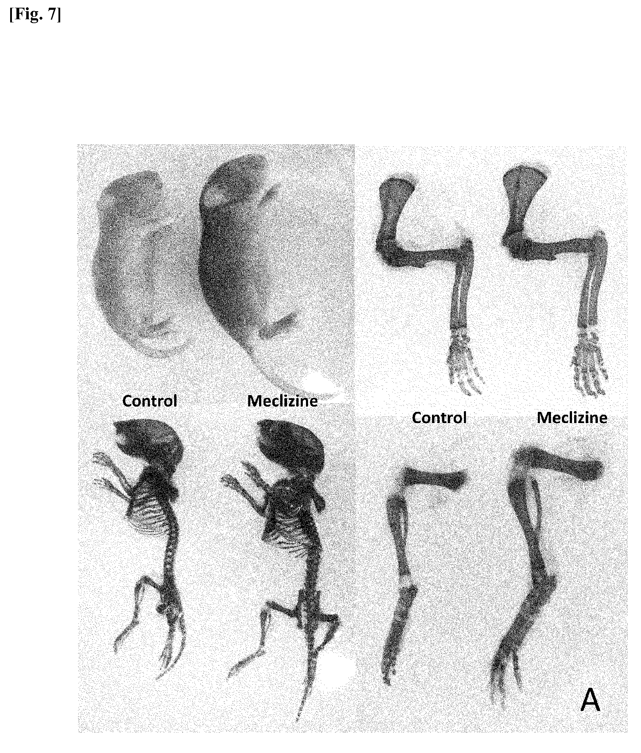

Fig. 7 shows the result of meclizine administration test. Meclizine was internally administered to a normal pregnant mouse from day 14 of pregnancy, and the body length and extremity length of the neonatal mouse on day 5 after birth were evaluated.

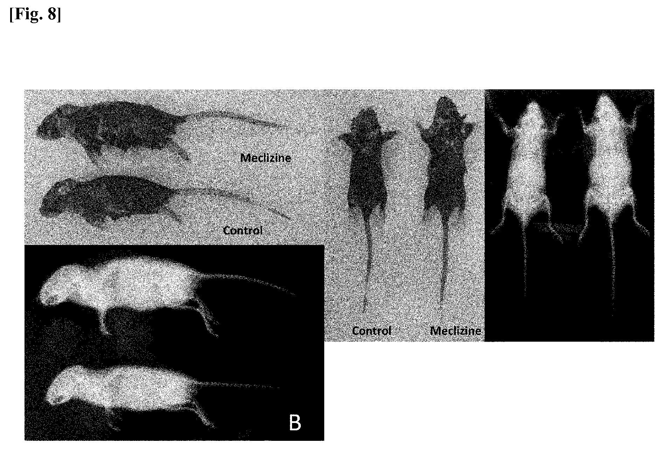

Fig. 8 shows the result of the meclizine administration test. Meclizine was internally administered to a normal mouse from week 2 after birth, and the body length and extremity length were evaluated on week 5.

### EMBODIMENT OF THE INVENTION

A first aspect of the present invention relates to a therapeutic agent for a skeletal dysplasia (hereinafter may be referred to as "the medicine of the present invention", for convenience of explanation) and the use thereof. The "therapeutic agent" means a medicine exhibiting therapeutic or prophylactic effect on the target disease or disease state. The therapeutic effect includes, for example, alleviation (relief) of the symptom or accompanying symptom characteristic to the target disease/disease state, and inhibition or delay of aggravation of the symptom. The latter can be regarded as a kind of prophylactic effect in terms of the prevention of aggravation. Thus the therapeutic effect and prophylactic effect are partly overlapping concepts, and it is difficult and slightly beneficial to clearly discriminate them. Typical prophylactic effect is the inhibition or delay of recurrence of the symptoms characteristic to the target disease/disease state. A drug falls into a therapeutic agent for the target disease/disease state as long as it shows therapeutic effect and/or prophylactic effect on the target disease/disease state.

The present invention is based on the result of finding of a novel pharmacological action of meclizine, more specifically the activity inhibiting FGFR3 signaling. The medicine of the present invention uses this pharmacological action, and is used in the treatment or prevention of a skeletal dysplasia caused by excessive activation of FGFR3. Although not wishing to be bound by any theory, the medicine of the present invention suppresses or inhibits excessive activation (in other words constant activation) of FGFR3, and thus achieves treatment effect. As shown by the below-described examples, meclizine was proved to show inhibition activity in the downstream of the classic RAS/MAPK cascade (signal transduction from MEK to ERK) (Fig. 6). This fact suggests that meclizine is an agent with high specificity and few side effects.

The "skeletal dysplasia" to be treated or prevented is not particularly limited as long as it is caused by excessive activation of FGFR3. In the present description, "caused by excessive activation of FGFR3" means that the excessive activation of FGFR3 is mainly or partly the cause of the disease. Examples of the applicable "skeletal dysplasia" include achondroplasia, hypochondroplasia, thanatophoric dysplasia, Crouzon disease, acromesomelic dysplasias, and severe achondroplasia with developmental delay and acanthosis nigricans (SADDAN). According to a preferred embodiment, the medicine of the present invention is used for treatment or prevention of achondroplasia or hypochondroplasia. Achondroplasia is a typical disease showing proximal short-limbed dwarfism, and occurs with high frequency. In most patients, G380R mutation in FGFR3 on the short arm of chromosome 4 (substitution of glycine at position 380 with arginine) is found. Because of the mutation, FGFR3 is constantly activated, and growth and differentiation of chondrocytes are inhibited. As a result of this, growth of the long bone in a longitudinal direction is markedly inhibited by endochondral ossification. Hypochondroplasia is also a disease showing proximal short-limbed dwarfism. The causal gene of hypochondroplasia is also FGFR3, and N540K mutation (substitution of asparagine at position 540 with ricin) is found in about half the patients. In the international classification of skeletal dysplasias, achondroplasia and hypochondroplasia are classified into the same group.

The medicine of the present invention contains meclizine or its pharmaceutically acceptable salt as an active ingredient. Meclizine is a compound having a substance name of 1-[(4-chloro phenyl)phenylmethyl]-4-[(3-methylphenyl)methyl] piperazine, and is known as an antihistamine. Meclizine is sold as an OTC (over the counter) motion sickness drug. Meclizine may be referred to as meclozine. In the present description, the terms "meclizine" and "meclozine" are interchangeable.

The active ingredient of the medicine of the present invention may be a pharmaceutically acceptable salt of meclizine. Commercially available meclizine preparations are mostly in the form of a hydrochloride of meclizine. Therefore, hydrochlorides are preferred in the present invention. However, the "pharmaceutically acceptable salt" will not be limited to them, and may be selected from various salts such as acid addition salts, metal salts, ammonium salts, organic amine addition salts, and amino acid addition salts. Examples of the acid addition salts include inorganic acid salts such as hydrochlorides, sulfates, nitrates, phosphates, and hydrobromides, and organic acid salts such as acetates, maleates, fumarates, citrates, benzenesulfonates, benzoates, malates, oxalates, methanesulfonates, and tartrates. Examples of the metal salts include alkali metal salts such as sodium salts, potassium salts, and lithium salts, alkaline earth metal salts such as magnesium salts and calcium salts, aluminum salts, and zinc salts. Examples of the ammonium salts include salts of ammonium and tetramethylammonium. Examples of the organic amine addition salts include morpholine addition salts and piperidine addition salts. Examples of the amino acid addition salts include glycine addition salts, phenylalanine addition salts, lysine addition salts, aspartic acid addition salts, and glutamic acid addition salts.

The formulation of the drug of the present invention may use a common proc edure. When formulated, other components which are acceptable for formulation (for example, a carrier, an excipient, a disintegrating agent, a buffering agent, an emulsifyi ng agent, a suspending agent, a soothing agent, a stabilizer, a preservative, an antisept ic, and a normal saline solution) may be added. Examples of the excipient include la ctose, starch, sorbitol, D-mannitol, and white sugar. Examples of the disintegrating ag ent include starch, carboxymethyl cellulose, and calcium carbonate. Examples of the buffering agent include phosphates, citrates, and acetates. Examples of the emulsifying agent include gum arabic, sodium alginate, and tragacanth. Examples of the suspendin g agent include glycerol monostearate, monostearic acid aluminum, methyl cellulose, ca rboxymethyl cellulose, hydroxymethyl cellulose, and sodium lauryl sulfate. Examples of the soothing agent include benzyl alcohol, chlorobutanol, and sorbitol. Examples o f the stabilizer include propylene glycol, and ascorbic acid. Examples of the preservat ive include phenol, benzalkonium chloride, benzyl alcohol, chlorobutanol, and methylpa raben. Examples of the antiseptic include benzalkonium chloride, paraoxybenzoic acid, and chlorobutanol.

The form of formulation is not particularly limited. Examples of the form in clude pellets, a powder, fine pellets, granules, capsules, a syrup, an injection, an exter nal preparation, and a suppository. The drug of the present invention is administered to the subject orally or parenterally (for example, intravenous, intraarterial, hypodermic, intradermal, intramuscular, or intraperitoneal injection, transdermal, nasotracheal, or tra nsmucosal administration). Systemic and local administrations are appropriately used a ccording to the subject. These administration routes are not exclusive each other, and optionally selected two or more may be combined (for example, intravenous injection or the like is carried out concurrently with oral administration or after a lapse of a predetermined period from finishing of oral administration). The drug of the present invention contains an active ingredient in an amount necessary for achieving the expe cted therapeutic effect (more specifically a therapeutically effective amount). The amo unt of the active ingredient in the drug of the present invention generally depends on the drug shape. In order to achieve the intended dose, the amount of the active ing redient is, for example, about 0.1% to 99% by weight.

The dose of the drug of the present invention is established to achieve the ex pected therapeutic effects. For the establishment of the therapeutically effective dose, in general, the symptoms, the age, sex, and body weight of the patient, and other fact ors are taken into consideration. Those skilled in the art can establish an appropriate dose in consideration of these factors. For example, the dose for an adult (body we ight: about 60 kg) may be established in such a manner that the amount of the active ingredient is 1 mg to 500 mg a day, preferably 5 mg to 300 mg a day, more prefer ably 10 mg to 200 mg a day. The administration schedule may be, for example, onc e to several times a day, once every two days, or once every three days. For the m aking of the administration schedule, the disease state of the patient, and the expected duration of the effect of the active ingredient may be taken into consideration. Exa mples of the method of local administration include the use during surgery, and injecti on into the intended location in the form of a carrier or any appropriate shape for the purpose of promoting the hearing process.

The treatment using the drug of the present invention may be carried out in p arallel with treatment using other drug (for example, an existing therapeutic drug). Al ternatively, an existing therapeutic procedure may be combined with the treatment usin g the drug of the present invention. Example of the existing therapies is a growth h ormone treatment. Example of existing therapeutic procedures is bone lengthening. B one lengthening uses a fixing device (internal or external fixing type) or a special dev ice called, for example, a bone lengthener. The method of bone lengthening usually i ncludes the processes of osteotomy, waiting period, bone lengthening period, and bone consolidation period. Details about bone lengthening are described in, for example, ADVANCE SERIES II-9, Hone Enchojutsu Saikin No Shinpo (Bone Lengthening: Rec ent advance (Kokuseido Co., Ltd., supervised by Kiyonori Harii, edited by Tsuneki Su gihara).

As is evident from the above-described explanation, the present application also provides a therapy including administering the medicine of the present invention to a patient with a skeletal dysplasia caused by excessive activation of FGFR3 in a pharmaceutically effective dose.

Another aspect of the present invention provides a body growth promoter including meclizine or its pharmaceutically acceptable salt as an active ingredient. The corresponding explanations in the above-described aspect are employed unless otherwise noted.

The body growth promoter of the present invention is applicable to healthy subjects, as well as patients with short stature. For example, the present invention is applicable to treatment or prevention of pituitary dwarfism not accompanied by epiphyseal closing, short stature in Turner syndrome, short stature in Prader-Willi syndrome, short stature with growth hormone deficiency (GHD), and short stature with small-for-gestational age (SGA). The body growth promoter of the present invention may be provided in the form of various compositions (for example, a pharmaceutical composition, quasi drug composition, or food composition).

When the body growth promoter of the present invention is provided in the form of a pharmaceutical composition or quasi drug composition, its formulation, dosage form, administration route, and dose are the same as those in the above-described aspect. On the other hand, when the body growth promoter of the present invention is provided in the form of a food composition, for example, it may be in the form of powder, granules, tablets, paste, or liquid of a dietary supplement (for example, a supplement or nutrition drink). The form of a food composition makes it easy to routinely or continuously take the body growth promoter of the present invention. The food composition of the present invention preferably contains an active ingredient in a therapeutically or prophylactically effective amount. The loading may be established in consideration of the disease condition, health condition, age, sex, and body weight of the subject.

### Examples

Drug repositioning strategy, in which a drug currently used for patients with a specific disease is applied to another disease, has gained increasing attention from both academia and industry in recent years (Reference 20, 21). The advantage of this strategy is that the identified drugs can be readily applied to clinical practice, because the optimal doses and adverse effects are already established. Here, we screened 1,186 FDA-approved compounds to identify a clinically applicable drug that ameliorates ACH/HCH and other FGFR3-related skeletal dysplasias.

### 1. MATERIALS AND METHODS

(1) Screening of 1,186 FDA-approved compounds in rat chondrosarcoma (RCS) cells
   RCS cells, which were kindly provided from Dr. Pavel Krejci (Medical Genetics Institute, Cedars-Sinai Medical Center, LA), were cultured in Dulbecco's Modified Eagle's Medium (DMEM, Invitrogen) supplemented with 10% fetal bovine serum (FBS, Thermo Scientific) (Reference 14). For the RCS growth arrest assays, ∼5 × 10³ cells were seeded in 96-well culture plates (Falcon) and incubated for 48 hours in the presence of 10 µM of 1,186 FDA-approved chemical compounds (Prestwick Chemical) and 5 ng/ml of FGF2 (R&D Systems). Cell proliferation was quantified by MTS assay (Cell 96 AQueus One Solution Cell Proliferation Assay, Promega) according to the manufacturer's instructions.
(2) Alcian blue staining
   For Alcian blue staining, growing RCS cells in 12-well plates were added with 5 ng/ml FGF2 and also with either 10 µM meclizine or 0.2 µM CNP (Calbiochem). After 72 hours, cells were fixed with methanol for 30 minutes at -20 °C, and stained overnight with 0.5% Alcian Blue 8 GX (Sigma) in 1 N HCl. For quantitative analyses, Alcian blue-stained cells were lysed in 200 µl of 6 M guanidine HCl for 6 hours at room temperature (Reference 28). The optical density of the extracted dye was measured at 610 nm using PowerScan 4 (DS Pharma Biomedical).
(3) Total RNA extraction and real-time RT-PCR analysis
   Total RNA was isolated from FGF2-treated RCS cells in the presence of 20 µM of meclizine or 0.2 µM of CNP using Trizol. The first strand cDNA was synthesized with ReverTra Ace (Toyobo). We quantified mRNA expression of matrix proteinases (*Mmp10, Mmp13,* and *Adamts1*) using LightCycler 480 Real-Time PCR (Roche) and SYBR Green (Takara). The mRNA levels were normalized for that of *Gapdh.*
(4) Vectors and cell transfection
   The pRK7-FGFR3-WT, -K650E, and -K650M vectors expressing wild-type and mutant FGFR3 (Reference 29) were kindly provided by Dr. Pavel Krejci (Medical Genetics Institute, Cedars-Sinai Medical Center, LA). The pRK7-FGFR3-G380R was constructed by the QuikChange site-directed mutagenesis kit (Stratagene). The wild-type and mutant *FGFR3* cDNAs were excised from pRK7-FGFR3 vectors by double digestion with *Hin*dIII and *Bam*HI*.* The fragments were cloned into the lentivirus vector, CSII-CMV-MCS-IRES2-Venus using the *Nhe*I and *Bam*HI sites. CSII-CMV-MCS-IRES2-Venus was kindly provided by Dr. Hiroyuki Miyoshi (Riken BioResource Center, Tsukuba, Japan.). The *Hin*dIII site of the insert and the *Nhe*I site of the vector were blunted using Quick Blunting Kit (New England Biolabs) before ligation. HEK293 cells were plated in 150-mm dishes the day before transfection. We introduced the pLP1, pLP2, pLP/VSVG plasmids (ViraPower Packaging Mix, Invitrogen), and the CSII-CMV-MCS-IRES2-Venus vector into HEK293 cells with Lipofectamine 2000 (Invitrogen) according to the manufacturer's protocols. At 48 hours after transfection, we filtered the media containing the virus particles using the Millex-HV 0.45 µm PVDF filters (Millex) and purified lentivirus using two steps of ultracentrifugation (Beckman Coulter). The lentivirus was added to the medium of HCS-2/8 or ATDC5 cells. After 48 hours, we confirmed that more than 90% of cells were positive for Venus signals.
   Clones that express constitutively active mutants in the MAPK/ERK pathway, pcDNA4Myc-ERK2(PD), pcDNA3HA-MEK1(DD), and pcDNA3Flag-C-rafΔN (Reference 30), were kindly provided by Dr. Mutsuhiro Takekawa (Medical Science Institute, Tokyo University, Japan). The inserts were digested with *Bam*HI and *Xho*I, and were cloned into the CSII-CMV-MCS-IRES2-Venus at the *Bam*HI sites after blunting all digested ends. Lentivirus particles were generated as described above and were applied to RCS cells.
(5) Growth assay of HCS-2/8 cells
   HCS-2/8 (Reference 31) cells were seeded in 96-well tissue culture plates. HCS-2/8 cells were then infected with lentivirus expressing either FGFR3-WT, -K650E, or -K650M. After 48 hours, the numbers of cells were estimated by MTS assay. In addition, ∼1 × 10⁵ HCS-2/8 cells in a 12-well tissue plate were introduced with lentivirus expressing FGFR3-K650E. After 72 hours, the fluorescence intensity of the Venus protein was quantified by the ArrayScan VTI HCS Reader (Thermo Scientific).
(6) Micromass culture of ATDC5 cells
   Mouse embryonic carcinoma-derived ATDC5 cells (Reference 32) were infected with either lentivirus expressing FGFR3-WT or FGFR3-G380R. The infected ATDC5 cells were subjected to micromass cultures as described previously (Reference 32). Briefly, ATDC5 cells were suspended in DMEM/F-12 (1:1) medium (Sigma) containing 5% FBS at a density of 1 × 10⁷ cells/ml and plated in 10-µl droplets to simulate the high-density chondrogenic condensations. After 1-hour incubation, the same medium supplemented with 1% insulin-transferrin-sodium selenite (ITS, Sigma) was added to the cells. Medium were changed every other day until harvesting on day 6.
(7) Bone explant culture
   For bone explant culture, tibiae were dissected under the microscope from wild-type mouse embryos on E16.5, placed in 96-well plates, and cultured in α-minimal essential medium (Invitrogen) supplemented with 0.2% bovine serum albumin, 1 mM β-glycerophosphate, and 50 µg/ml ascorbic acid. Embryonic tibiae were further treated with 100 ng/ml FGF2 in the presence or absence of 20 µM meclizine or 0.2 µM CNP for 6 days, then fixed in 10% formaldehyde in phosphate-buffered saline, demineralized with 0.5 M EDTA, and embedded in paraffin. Sections were stained with hematoxylin- eosin and Alcian blue. Images were taken with the SZ61 microscope (Olympus) equipped with the XZ-1 digital camera (Olympus). The longitudinal length of bone, which was defined as the length between proximal and distal articular cartilage, was measured using ImageJ (NIH).
(8) Western blotting and signaling studies
   RCS cells were treated with 20 µM meclizine for 30 min before adding 5 ng/ml FGF2 ("Vehicle" is no treatment group). After 5 min, cells were lysed in the ice-cold RIPA Lysis Buffer (Santa Cruz) supplemented with proteinase inhibitors. Whole cell lysates were separated on SDS-PAGE and transferred to a nitrocellulose membrane. The phosphorylation levels of molecules in the MAPK pathway were determined by Western blotting using the following antibodies: ERK1/2, phospho-ERK1/2(Thr²⁰²/Tyr²⁰⁴), MEK1/2, and phospho-MEK1/2(Ser^{217/221}) (Cell Signaling).
(9) Internal administration of meclizine to normal mouse
   Meclizine was internally administered to a normal pregnant mouse from day 14 of pregnancy (2 mg of meclizine is contained in 5 g of feed), and the body length and extremity length of the neonatal mouse on day 5 after birth were evaluated. On the other hand, meclizine was internally administered to a normal mouse from week 2 after birth, and the mouse was evaluated in the same manner on week 5.

### 2. RESULTS

(1) Meclizine promotes chondrocyte proliferation and rescues loss of extracellular matrix in FGF2-treated RCS cells
   As RCS chondrocytic cells express high levels of FGFR3, exogenous administration of FGF2 readily recapitulates cellular processes occurring in FGFR3-related skeletal dysplasias (Reference 22). We thus added 10 µM of 1,186 FDA-approved chemical compounds (Prestwick Chemical) along with 5 ng/ml FGF2 to the RCS culture medium. Quantification of RCS proliferation by MTS assay revealed that meclizine consistently induced 1.4-fold or more increases in RCS proliferation. In addition, 0, 1, 2, 5, 10, 20, and 50 µM of meclizine exhibited dose-dependent increases in RCS proliferation, although the cell toxicity was evident at 50 µM (Fig. 1A).
   We next compared the meclizine effect with that of CNP (Reference 6, 17) as a positive control. Growing RCS cells produce a large amount of cartilage-like sulfated proteoglycans, which can be visualized by Alcian blue staining. The matrix proteoglycans are almost completely lost at 72 hours after addition of FGF2 due to inhibition of proteoglycan production as well as to induction of matrix metalloproteinase-mediated degradation (Reference 6). In the cells treated with FGF2 together with meclizine, staining with Alcian blue as well as round chondrocyte-like cell shapes were restored, which was similar to those observed in the CNP-treated cells (Fig. 1B). RCS cells treated with FGF2 for four hours increase expressions of *Mmp10, Mmp13,* and *Adamts1* (Reference 6). We also confirmed that FGF2 induces expressions of *Mmp10, Mmp13,* and *Adamts1* in RCS cells, and found that meclizine and CNP significantly suppressed expressions of these matrix metalloproteinases (Fig. 1C). These results suggest that meclizine rescues loss of matrix proteoglycans by decreasing expressions of the matrix metalloproteinases in FGF2-treated RCS cells.
(2) Meclizine rescues abnormally suppressed proliferation of HCS-2/8 chondrocytes expressing FGFR3-K650E and -K650M
   We next introduced lentivirus carrying activated mutants of FGFR3 (K650E in TD II and K650M in SADDAN) into human chondrosarcoma (HCS-2/8) cells to evaluate the effect of meclizine on inhibition of the constitutively active FGFR3 signaling. MTS assay demonstrated that K650E-expressing HCS-2/8 showed significantly suppressed cellular proliferation (Fig. 2A). Meclizine (20 µM) partially but significantly rescued the growth arrest without apparent cellular toxicity in both K650E- and K650M-expressing HCS-2/8 (Fig. 2B). Integrated intensities of Venus signals, which represent the signal intensities multiplied by the number of Venus-positive cells, were significantly increased in the meclizine-treated K650E-expressing HCS-2/8 (Fig. 2C).
(3) Meclizine rescues abnormal inhibited differentiation of ATDC5 chondrogenic cells expressing FGFR3-G380R
   ATDC5 cells retain potency to differentiate into chondrocytes and are commonly used to study chondrogenesis *in vitro* (Reference 15). To determine whether meclizine rescues suppression of chondrogenesis mediated by mutant FGFR3, we added 20 µM meclizine to the micromass culture of ATDC5 cells that were infected with lentivirus expressing FGFR3-G380R causing ACH. Alcian blue staining grossly revealed abundant sulfated proteoglycans on wild-type FGFR3 cells, while the staining intensity was reduced on FGFR3-G380R cells. Addition of meclizine from the beginning of the chondrogenic induction significantly reversed the inhibitory effect of FGFR3-G380R on chondrogenesis. Quantitative analysis of sulfated glycosaminoglycans by measuring 610 nm absorbance of cell lysate with guanidine HCl demonstrated that meclizine significantly increased the levels of glycosaminoglycans in FGFR3-G380R cells, which underscored the morphological observations with Alcian blue staining (Fig. 3). These findings suggest that meclizine rescues the inhibitory effect of mutant FGFR3 on chondrocyte differentiation.
(4) Meclizine increases the longitudinal length of embryonic tibiae with or without FGF2 treatment in bone explant culture
   We next examined the effect of meclizine on FGF2-mediated inhibition of cartilage development in the bone organ culture employing limb rudiments isolated from developing murine embryonic tibia (Reference 14). We added CNP(0.2 µM) or meclizine(20 µM) together with FGF2(100 ng/ml) to the culture medium, and compared the length of treated tibiae with that of contralateral control tibia from the same individual. Addition of FGF2 inhibited longitudinal bone and cartilage growth, while CNP and meclizine significantly attenuated FGF2-mediated growth inhibition of embryonic tibiae on day 6 (Fig. 4). It is interesting to note that meclizine also increased the length of tibia without FGF2 treatment.
(5) Meclizine attenuates ERK phosphorylation in FGF2-treated RCS cells
   We next examined the effects of meclizine on downstream signaling of FGFR3 in FGF2-treated RCS cells. RCS cells were pretreated with meclizine for 30 minutes before adding FGF2, and the phosphorylation levels of ERK and MEK were determined by Western blotting. The FGF2-mediated ERK1/2 phosphorylation was attenuated by meclizine, while MEK1/2 phosphorylation remained unchanged (Fig. 5A). We next introduced constitutively active (ca) mutants of ERK, MEK, and RAF into RCS cells using lentivirus and quantified cell growth with MTS assay. As predicted, meclizine rescued caMEK- and caRAF-mediated growth inhibition, whereas meclizine had no effect on caERK-mediated growth inhibition (Fig. 5B). Both data point to a notion that meclizine either inhibits phosphorylated MEK-mediated ERK phosphorylation or activates phosphatase(s) for phosphorylated ERK (Fig. 6).
(6) Meclizine increases body length and extremity length
   Meclizine was internally administered to a normal pregnant mouse from day 14 of pregnancy, and the neonatal mouse on day 5 after birth was evaluated; the increase of the body length, superior and inferior limbs was found in the meclizine administration group (Fig. 7). In addition, meclizine was administered to a normal mouse from week 2 after birth, and evaluated on 5 week after birth; the increase of the body length and tail length was found in the meclizine administration group (Fig. 8).

### DISCUSSION

Drug repositioning strategy is an effort to identify new indications for the existing drugs. This strategy can potentially avoid costs and time associated with multi-stage testing of the hit compounds (Reference 20, 23). Among 1,186 FDA-approved drugs that have favorable or validated pharmacokinetic properties and toxicological profiles, we identified meclizine as a novel inhibitor of FGFR3 signaling, which can be potentially applied to clinical practice for short stature of skeletal dysplasias (ACH/HCH). Meclizine is an Over-the-Counter (OTC) H1 blocker, which has been safely used for motion sickness for more than 50 years. Because the optimal doses and adverse effects of meclizine have already been established, meclizine can be readily prescribed after an inhibitory effect on FGFR3 in an appropriate concentration for clinical application has been confirmed.

Since there is no rational therapy for ACH/HCH available to date, development of novel modalities to suppress FGFR3 signaling has long been expected. Krejci et al. screened a library of 1,120 compounds and identified that NF449 inhibits FGFR3 signaling in RCS chondrocytes as well as in FGF2-treated embryonic bone organ culture. NF449 is structurally similar to suramin and possesses inhibitory activities of other tyrosine kinases in addition to FGFR3 (Reference 14). Jonquoyet et al. identified that a synthetic compound A31 is an inhibitor of FGFR3 tyrosine kinase by *in silico* analysis. They demonstrated that A31 suppresses constitutive phosphorylation of FGFR3 and restores the size of embryonic femurs of *Fgfr3*^{Y361C/+} mice in organ culture. In addition, A31 potentiates chondrocytes differentiation in the *Fgfr3*^{Y367C/+} growth plate (Reference 16). Jin et al. screened a library of random 12-peptide phages and found that P3 has a high and specific binding affinity for the extracellular domain of FGFR3. They showed that P3 promotes proliferation and chondrogenic differentiation of cultured ATDC5 cells, alleviates the bone growth retardation in bone rudiments from TD mice (*Fgfr3*^{Neo-K644E/+} mice), and finally reversed the neonatal lethality of TD mice (Reference 15). These novel FGFR3 tyrosine kinase inhibitors, however, may inhibit tyrosine kinases other than FGFR3 and may exert unexpected toxic effects to humans. Meclizine may also inhibit unpredicted tyrosine kinases, but we can predict that there will be no overt adverse effect, because meclizine has been safely prescribed for more than 50 years.

CNP is another therapeutic agent for achondroplasia. CNP-deficient mice were dwarfed with narrowing of the proliferative and hypertrophic zone of the growth plates (Reference 24). Loss of function mutations in *NPR2* encoding a receptor for CNP are responsible for short-limbed human skeletal dysplasia, acromesomelic dysplasia Maroteaux-type (AMDM) (Reference 25). Conversely, overexpression of CNP prevented the shortening of achondroplastic bones by inhibiting the MAPK signaling pathway (Reference 17). Yasoda et al. demonstrated that continuous delivery of CNP through intravenous infusion successfully normalized the dwarfism of *Fgfr3*^{ach} mice. CNP, however, was required to be continuously infused because of its very short half-life (Reference 18). Lorget et al. demonstrated that a CNP analog, BMN111, has an extended plasma half-life due to its resistance to neutral-endopeptidase digestion, and is capable of leading to a significant recovery of bone growth in *Fgfr3*^{Y367C/+} mice by subcutaneous administration (Reference 19). Meclizine showed a similar inhibitory activity on the FGFR3 signaling compared to CNP in *ex vivo* bone explant culture as well as *in vitro* chondrogenic cells. As meclizine has long been used in clinical practice for motion sickness, we expect that meclizine can be used as a substitute for CNP and CNP-analog.

The MAPK pathway is one of the major pathways downstream of the FGFR3 signaling in proliferation and differentiation of chondrocytes. Sustained ERK activation in chondrocytes leads to decreased proliferation, increased matrix degradation, altered cell shape, and decreased differentiation (Reference 5, 6). CNP inhibits phosphorylation of RAF1 kinase through inhibition by PKGII (Reference 6, 17). We demonstrated that meclizine attenuates ERK phosphorylation in chondrocytes. Gohil et al. reported that meclizine has an anti-oxidative phosphorylation (OXPHOS) activity in addition to anti-histamine and anti-muscarinic properties (Reference 26, 27). In their report, meclizine showed cytoprotective activities against ischemic injury in the brain and heart. Since other drugs with anti-histamine, anti-muscarinic, and anti-OXPHOS properties did not show inhibition of FGFR3 signaling in our studies, pharmacological actions of meclizine on chondrogenesis are unlikely to be relevant to its anti-histamine, anti-muscarinic, or anti-OXPHOS properties.

In the current studies, we have demonstrated that meclizine rescues growth arrest, loss of extracellular matrix, dysmorphology of cellular shape, and matrix degradation of FGF2-treated RCS chondrocytes. Meclizine also rescued the impaired proliferation and differentiation of HCS and ATDC5 cells expressing mutant FGFR3. In addition, meclizine rescued FGF2-mediated growth inhibition in limb rudiments. Meclizine is an attractive and feasible therapeutic modality to treat ACH/HCH.

On the other hand, the result of the animal experiment supported that meclizine has extension effect on the body length and extremity. In view of this fact, for example, meclizine is considered effective also for pituitary dwarfism not accompanied by epiphyseal closing, short stature in Turner syndrome, short stature in Prader-Willi syndrome, short stature with growth hormone deficiency (GHD), and short stature with small-for-gestational age (SGA).

### INDUSTRIAL APPLICABILITY

The therapeutic agent of the present invention shows its efficacy by the novel action of meclizine, or the suppression of FGFR3 signaling. Meclizine is sold as an OTC (over the counter) motion sickness drug which has been used safely over 50 years. Therefore, its safety including the optimal dose, side effect, and contraindication is established. This fact is markedly advantageous in the clinical application. The present invention is expected to be applied to various skeletal dysplasias caused by excessive activation of FGFR3, such as achondroplasia, hypochondroplasia, thanatophoric dysplasia, Crouzon disease, acromesomelic dysplasias, and severe achondroplasia with developmental delay and acanthosis nigricans (SADDAN).

The present invention will not be limited to the description of the embodimen ts and examples of the present invention. Various modifications readily made by thos e skilled in the art are also included in the present invention, without departing from the scope of claims. The entire contents of the articles, unexamined patent publicatio ns, and patent applications specified herein are hereby incorporated herein by reference.

### <References>

1. Waller, D.K., Correa, A., Vo, T.M., Wang, Y., Hobbs, C., Langlois, P.H., Pearson, K., Romitti, P.A., Shaw, G.M. and Hecht, J.T. (2008) The population-based prevalence of acho ndroplasia and thanatophoric dysplasia in selected regions of the US. Am J Med Genet A, 146 A, 2385-2389.
2. Horton, W.A., Hall, J.G. and Hecht, J.T. (2007) Achondroplasia. Lancet, 370, 162-172.
3. Rousseau, F., Bonaventure, J., Legeai-Mallet, L., Pelet, A., Rozet, J.M., Maroteaux , P., Le Merrer, M. and Munnich, A. (1994) Mutations in the gene encoding fibroblast growth factor receptor-3 in achondroplasia. Nature, 371, 252-254.
4. Shiang, R., Thompson, L.M., Zhu, Y.Z., Church, D.M., Fielder, T.J., Bocian, M., Winokur, S.T. and Wasmuth, J.J. (1994) Mutations in the transmembrane domain of FGFR3 c ause the most common genetic form of dwarfism, achondroplasia. Cell, 78, 335-342.
5. Krejci, P., Bryja, V., Pachernik, J., Hampl, A., Pogue, R., Mekikian, P. and Wilcox, W.R. (2004) FGF2 inhibits proliferation and alters the cartilage-like phenotype of RCS cells. Exp. Cell Res., 297, 152-164.
6. Krejci, P., Masri, B., Fontaine, V., Mekikian, P.B., Weis, M., Prats, H. and Wilcox, W.R. (2005) Interaction of fibroblast growth factor and C-natriuretic peptide signaling in regu lation of chondrocyte proliferation and extracellular matrix homeostasis. J. Cell Sci., 118, 508 9-5100.
7. Murakami, S., Balmes, G., McKinney, S., Zhang, Z., Givol, D. and de Crombruggh e, B. (2004) Constitutive activation of MEK1 in chondrocytes causes Stat1-independent achon droplasia-like dwarfism and rescues the Fgfr3-deficient mouse phenotype. Genes Dev., 18, 29 0-305.
8. Prinos, P., Costa, T., Sommer, A., Kilpatrick, M.W. and Tsipouras, P. (1995) A com mon FGFR3 gene mutation in hypochondroplasia. Hum. Mol. Genet., 4, 2097-2101.
9. Tavormina, P.L., Bellus, G.A., Webster, M.K., Bamshad, M.J., Fraley, A.E., McInto sh, I., Szabo, J., Jiang, W., Jabs, E.W., Wilcox, W.R. et al. (1999) A novel skeletal dysplasia wi th developmental delay and acanthosis nigricans is caused by a Lys650Met mutation in the fib roblast growth factor receptor 3 gene. Am J Hum Genet, 64, 722-731.
10. Tavormina, P.L., Shiang, R., Thompson, L.M., Zhu, Y.Z., Wilkin, D.J., Lachman, R.S., Wilcox, W.R., Rimoin, D.L., Cohn, D.H. and Wasmuth, J.J. (1995) Thanatophoric dyspl asia (types I and II) caused by distinct mutations in fibroblast growth factor receptor 3. Nat. G enet., 9, 321-328.
11. Toydemir, R.M., Brassington, A.E., Bayrak-Toydemir, P., Krakowiak, P.A., Jorde, L.B., Whitby, F.G., Longo, N., Viskochil, D.H., Carey, J.C. and Bamshad, M.J. (2006) A nove 1 mutation in FGFR3 causes camptodactyly, tall stature, and hearing loss (CATSHL) syndrom e. Am J Hum Genet, 79, 935-941.
12. Beever, J.E., Smit, M.A., Meyers, S.N., Hadfield, T.S., Bottema, C., Albretsen, J. and Cockett, N.E. (2006) A single-base change in the tyrosine kinase II domain of ovine FGF R3 causes hereditary chondrodysplasia in sheep. Anim. Genet., 37, 66-71.
13. Horton, W.A., Hecht, J.T., Hood, O.J., Marshall, R.N., Moore, W.V. and Hollowel l, J.G. (1992) Growth hormone therapy in achondroplasia. Am. J. Med. Genet., 42, 667-670.
14. Krejci, P., Murakami, S., Prochazkova, J., Trantirek, L., Chlebova, K., Ouyang, Z ., Aklian, A., Smutny, J., Bryja, V., Kozubik, A. et al. (2010) NF449 is a novel inhibitor of fibr oblast growth factor receptor 3 (FGFR3) signaling active in chondrocytes and multiple myelo ma cells. J. Biol. Chem., 285, 20644-20653.
15. Jin, M., Yu, Y., Qi, H., Xie, Y., Su, N., Wang, X., Tan, Q., Luo, F., Zhu, Y., Wang, Q. et al. (2012) A novel FGFR3-binding peptide inhibits FGFR3 signaling and reverses the let hal phenotype of mice mimicking human thanatophoric dysplasia. Hum. Mol. Genet., 21, 544 3-5455.
16. Jonquoy, A., Mugniery, E., Benoist-Lasselin, C., Kaci, N., Le Corre, L., Barbault, F., Girard, A.L., Le Merrer, Y., Busca, P., Schibler, L. et al. (2012) A novel tyrosine kinase inh ibitor restores chondrocyte differentiation and promotes bone growth in a gain-of-function Fgf r3 mouse model. Hum. Mol. Genet., 21, 841-851.
17. Yasoda, A., Komatsu, Y., Chusho, H., Miyazawa, T., Ozasa, A., Miura, M., Kurih ara, T., Rogi, T., Tanaka, S., Suda, M. et al. (2004) Overexpression of CNP in chondrocytes re scues achondroplasia through a MAPK-dependent pathway. Nat. Med., 10, 80-86.
18. Yasoda, A., Kitamura, H., Fujii, T., Kondo, E., Murao, N., Miura, M., Kanamoto, N., Komatsu, Y., Arai, H. and Nakao, K. (2009) Systemic administration of C-type natriuretic peptide as a novel therapeutic strategy for skeletal dysplasias. Endocrinology, 150, 3138-3144
19. Lorget, F., Kaci, N., Peng, J., Benoist-Lasselin, C., Mugniery, E., Oppeneer, T., W endt, D.J., Bell, S.M., Bullens, S., Bunting, S. et al. (2012) Evaluation of the therapeutic poten tial of a CNP analog in a Fgfr3 mouse model recapitulating achondroplasia. Am J Hum Genet, 91, 1108-1114.
20. Abbott, A. (2002) Neurologists strike gold in drug screen effort. Nature, 417, 109.
21. Bian, Y., Masuda, A., Matsuura, T., Ito, M., Okushin, K., Engel, A.G. and Ohno, K. (2009) Tannic acid facilitates expression of the polypyrimidine tract binding protein and all eviates deleterious inclusion of CHRNA1 exon P3A due to an hnRNP H-disrupting mutation i n congenital myasthenic syndrome. Hum. Mol. Genet., 18, 1229-1237.
22. Dailey, L., Laplantine, E., Priore, R. and Basilico, C. (2003) A network of transcri ptional and signaling events is activated by FGF to induce chondrocyte growth arrest and diff erentiation. J. Cell Biol., 161, 1053-1066.
23. Heemskerk, J., Tobin, A.J. and Bain, L.J. (2002) Teaching old drugs new tricks. Meeting of the Neurodegeneration Drug Screening Consortium, 7-8 April 2002, Washington, DC, USA. Trends Neurosci., 25, 494-496.
24. Chusho, H., Tamura, N., Ogawa, Y., Yasoda, A., Suda, M., Miyazawa, T., Nakam ura, K., Nakao, K., Kurihara, T., Komatsu, Y et al. (2001) Dwarfism and early death in mice 1 acking C-type natriuretic peptide. Proc. Natl. Acad. Sci. U. S. A., 98, 4016-4021.
25. Bartels, C.F., Bukulmez, H., Padayatti, P., Rhee, D.K., van Ravenswaaij-Arts, C., Pauli, R.M., Mundlos, S., Chitayat, D., Shih, L.Y., Al-Gazali, L.I. et al. (2004) Mutations in th e transmembrane natriuretic peptide receptor NPR-B impair skeletal growth and cause acrome somelic dysplasia, type Maroteaux. Am J Hum Genet, 75, 27-34.
26. Gohil, V.M., Sheth, S.A., Nilsson, R., Wojtovich, A.P., Lee, J.H., Perocchi, F., Ch en, W., Clish, C.B., Ayata, C., Brookes, P.S. et al. (2010) Nutrient-sensitized screening for dru gs that shift energy metabolism from mitochondrial respiration to glycolysis. Nat. Biotechnol., 28, 249-255.
27. Gohil, V.M., Offner, N., Walker, J.A., Sheth, S.A., Fossale, E., Gusella, J.F., Mac Donald, M.E., Neri, C. and Mootha, V.K. (2011) Meclizine is neuroprotective in models of Hu ntington's disease. Hum. Mol. Genet., 20, 294-300.
28. De Bari, C., Dell'Accio, F. and Luyten, F.P. (2001) Human periosteum-derived ce lls maintain phenotypic stability and chondrogenic potential throughout expansion regardless of donor age. Arthritis Rheum., 44, 85-95.
29. Krejci, P., Masri, B., Salazar, L., Farrington-Rock, C., Prats, H., Thompson, L.M. and Wilcox, W.R. (2007) Bisindolylmaleimide I suppresses fibroblast growth factor-mediated activation of Erk MAP kinase in chondrocytes by preventing Shp2 association with the Frs2 a nd Gab1 adaptor proteins. J. Biol. Chem., 282, 2929-2936.
30. Emrick, M.A., Hoofnagle, A.N., Miller, A.S., Ten Eyck, L.F. and Ahn, N.G. (2001 ) Constitutive activation of extracellular signal-regulated kinase 2 by synergistic point mutatio ns. J. Biol. Chem., 276, 46469-46479.
31. Takigawa, M., Tajima, K., Pan, H.O., Enomoto, M., Kinoshita, A., Suzuki, F., Tak ano, Y. and Mori, Y. (1989) Establishment of a clonal human chondrosarcoma cell line with ca rtilage phenotypes. Cancer Res., 49, 3996-4002.
32. Atsumi, T., Miwa, Y., Kimata, K. and Ikawa, Y (1990) A chondrogenic cell line d erived from a differentiating culture of AT805 teratocarcinoma cells. Cell Differ. Dev., 30, 109 -116.
33. Hoogendam, J., Parlevliet, E., Miclea, R., Lowik, C.W., Wit, J.M. and Karperien, M. (2006) Novel early target genes of parathyroid hormone-related peptide in chondrocytes. E ndocrinology, 147, 3141-3152.

## Claims

1. A therapeutic agent for a skeletal dysplasia caused by excessive activation of fibroblast growth factor receptor 3 (FGFR3), the therapeutic agent comprising meclizine or its pharmaceutically acceptable salt as an active ingredient.

2. The therapeutic agent of claim 1, wherein the skeletal dysplasia is a disease selected from the group consisting of achondroplasia, hypochondroplasia, thanatophoric dysplasia, Crouzon disease, acromesomelic dysplasias, and severe achondroplasia with developmental delay and acanthosis nigricans (SADDAN).

3. The therapeutic agent of claim 1 or 2, wherein the active ingredient is meclizine hydrochloride.

4. A therapy for a skeletal dysplasia, comprising administering meclizine or its pharmaceutically acceptable salt to a patient with a skeletal dysplasia caused by excessive activation of fibroblast growth factor receptor 3 (FGFR3) in a pharmaceutically effective dose.

5. A body growth promoter comprising meclizine or its pharmaceutically acceptable salt as an active ingredient.

6. A body growth promoting composition comprising the body growth promoter of claim 5.

7. The body growth promoting composition of claim 6, which is a medicine, quasi drug, or food.
